Europäisches Patentamt

European Patent Office

Office européen des brevets

(11) Publication number: **0 353 780**
**A2**

## EUROPEAN PATENT APPLICATION

(21) Application number: 89114485.9

(22) Date of filing: 04.08.89

(51) Int. Cl.4: **C07K 15/08 , C07K 3/20 ,**
**C07K 3/22 , A61K 37/02 ,**
**A61K 35/60**

Claims for the following Contracting States: ES + GR.

(30) Priority: 04.08.88 IL 87349

(43) Date of publication of application:
07.02.90 Bulletin 90/06

(84) Designated Contracting States:
AT BE CH DE ES FR GB GR IT LI LU NL SE

(71) Applicant: YEDA RESEARCH & DEVELOPMENT
COMPANY, LIMITED
P.O. Box 95
Rehovot 76100(IL)

(72) Inventor: Cohen, Avi
29/4 Eisenberg Street
Rehovot(IL)
Inventor: Belkin, Michael
4, Alonim Street
Givat Shmuel(IL)
Inventor: Schwartz, Michal
Neve Metz 10 Weizmann Institute of Science
Rehovot(IL)

(74) Representative: Vossius & Partner
Siebertstrasse 4 P.O. Box 86 07 67
D-8000 München 86(DE)

(54) **Factor for the activation of target cells.**

(57) A water soluble factor is derived from regenerating nerves, such as the optical nerves of fish, and this factor activates suitable target cells, such as for example glial cells, to produce extracellular proteins. Mainly laminin is thus produced. The factor is a negatively charged protein which has an apparent molecular weight of about 10,000 daltons, determined on a sieving type column. There is also provided a process for the purification of such factor. Furthermore there are provided pharmaceutical compositions which induce regeneration of nerves of the central nervous system of mammals, which contain as actaive ingredient an effective quantity of such purified facator. The effect of this factor on the regeneration of nerves is augmented when the injured site is irradiated with radiation from a low energy He-Ne laser.

# FACTOR FOR THE ACTIVATION OF TARGET CELLS

The invention relates to a purified water-soluble factor which is obtainable from the regenerating optic nerve of a fish, and which activates suitable target cells, such as glial cells, to produce certain extracellular proteins, and mainly laminin.

The invention further relates to pharmaceutical compositions containing such a purified factor as active ingredient. Furthermore, the invention relates to a process for the purification of such a factor from a natural source such as a regenerating optic nerve of a fish.

Adult injured optic nerves of mammals show an increased distribution of laminin. in the extracellular matrix after application of soluble substances in the form of conditioned media originating from growing nerves (Zak, N., Harel, A , Bawnik, Y., Benbassat, S., Vogel, Z. and Schwartz, M. Brain Res. 408 (1987) 263-266). This increase could result from a direct or an indirect activation of glial cells, or from an activation of other cellular elements. Laminin is known to be a key macromolecule for supporting growth and elongation of central and peripheral nervous system neurons (CNS and PNS, respectively), both in vivo and in vitro (Carbonetto, S., Gruver, M.M. and Turner, D.C. J. Neurosci. 3 (1983) 2324-2335; Davis, G.E., Blaker, S.N., Engvall, E., Varon, S., Manthorpe, M. and Gage, F.H. Science 236 (1987) 1106-1110; Hopkins, J.M., Ford-Holevinski, T.S., McCoy, J.P., Agranoff, B.W. J. Neurosci. 5 (1985) 3030-3038; Longo, F.M., Hayman, E.G., Davis, G.E., Ruoslahti, E., Engvall, E., Manthorpe, M. and Varon, S. Brain Res. 309 (1984) 105-112; Manthorpe, M., Engvall, E., Ruoslahti, E., Longo, F.M., Davis, G.E. and Varon, S. J. Cell Biol. 97 (1983) 1882-1890; Smalheiser, N.R., Crain, S.M. and Reid, L.M. Dev. Brain Res. 12 (1984) 135-140; Williams, L.R. and Varon, S. J. Comp. Neurolog. 231 (1985) 209-220). Laminin is absent in CNS of adult mammals and this might be a reason for the poor regenerative ability of mammalian CNS. We have shown that the deficiency in laminin may be reversed by in situ application of conditioned media from regenerating fish optic nerves (Schwartz, M., Belkin, M., Harel, A., Solomon, A., Lavie, V., Hadani, M., Rachailovich, I. and Stein-Izsak, C. Science 228 (1986) 600-603; Zak, N., Harel, A., Bawnik, Y., Benbassat, S., Vogel, Z. and Schwartz, M. Brain Res. 408 (1987) 263-266). It was shown that laminin production by glial cells is correlated with the growth state of the nerve (Hopkins, J.M., Ford-Holevinski, T.S., McCoy, J.P. and Agranoff, B.W. J. Neurosci. 5 (1985) 3030-3038; Liesi, P. EMBO J. 4 (1985) 2505-2511).

The present invention relates to a water soluble factor which is obtainable from a regenerating optic nerve of a fish which activates suitable target cells, such as glial cells, to produce extracellular proteins, mainly laminin. This is a factor which modifies glial cell response to injury in the mammalian central nervous system and enhances protein synthesis, mainly that of laminin. The factor is a negatively charged protein which exhibits one peak of an apparent molecular weight of about 10,000 and which when subjected to chromatography on a sieving type column of the Mono-Q type, exhibits two peaks, one of which is eluted at 0.16 M sodium chloride, the other at about 0.3 M sodium chloride.

Furthermore, the invention relates to a pharmaceutical composition for inducing the regeneration of nerves of the central nerve system of mammals which contains as active ingredient an effective quantity of a factor as defined above. The invention also relates to a process for the separation and purification of a factor which can induce laminin production and accumulation which comprises subjecting materials released from regenerating optical nerves of fish to a separation process on columns specified herein, and eluting such factor by suitable eluants.

Furthermore the invention relates to a method of inducing regeneration of injured nerves of the mammalian central nerve system, which comprises applying to the injured nerve an effective quantity of a factor defined above. According to a preferred embodiment in addition to the application of the factor, the injured site is irradiated with a low energy He-Ne laser at predetermined intervals, the site is advantageously irradiated daily.

In the following description the following abbreviations are used: Central nervous system - CNS; conditioned media -CM; fetal calf serum - FCS; sodium dodecyl sulfate poly acrylamide gel electrophoresis - SDS-PAGE.

The invention is further illustrated with reference to the enclosed Figures in which:

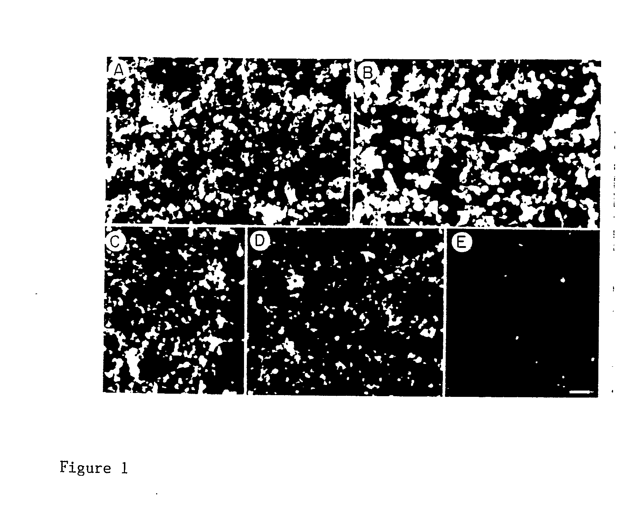

Fig. 1 illustrates the laminin immunofluorescent sites in cells of C-6 glioma cell line. Cells were grown in Chamber slide in DMEM containing 10% FCS till they reached confluency. The cells were then washed and incubated for 24 h in Waymouth medium supplemented with insulin and bovine serum albumin. After 24 h, the various plates were further treated with CM or with FCS in the supplemented Waymouth medium. After additional 24 h the medium was collected, cells were washed (x3) in Tris buffer (10 mM Tris 7.4, 150 mM NaCl) and then fixed for 15 min in ethanol (96%) followed by wash in Tris buffer. Reaction with the primary antibodies was carried out for 45 min at 37°C, followed by wash (x3) in Tris buffer and then for 45 min in FITC conjugated to goat anti-rabbit IgG (1:100) followed by 3 washes in Tris buffer. Samples were then coated with a solution of glycerol (90% in PBS) and covered with glass.

A. Cells treated with 1 μg/ml CM; B. Cells treated with 0.1 μg/ml CM; C. Cells treated with 10% FCS; D. Cells kept in Waymouth free of serum or conditioned medium. All groups were stained with anti-laminin antibodies, except for one group shown in photograph E, which represents cells as in A but the incubation with anti-laminin antibodies was omitted and replaced by incubation with rabbit preimmune sera.

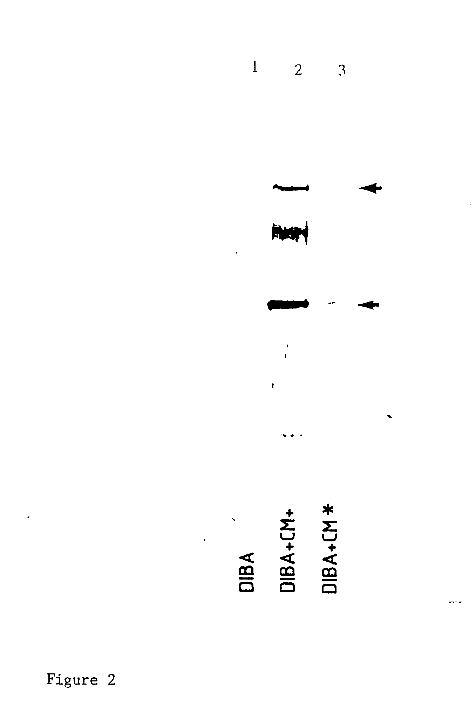

Fig. 2 illustrates the increased labeling of laminin in CM-treated cells. Cells were treated as in Figure 1. The last 24 h of incubation was carried out in medium which was supplemented also with [$^{35}$S]-methionine (12 μCi; 113 uCi/mmole, Amersham). Laminin containing fraction was obtained by following the protocol previously reported by Clark et al. (1985). Cells were labeled with [$^{35}$S]-methionine. Following labeling, the media in which the cells were grown and the cells were collected separately. The cells were homogenized in buffer containing detergent (10 mM Tris-Cl pH 7.4; 150 mM NaCl; 1% Triton X100; 1 mM EDTA, 20 μg/ml pepstatin; 1 mM benzamidine; 20 U/ml aproptonin; 1 mM PMSF; 20 ug/ml leupeptin). The soluble double fraction obtained after centrifugation at 27,000 xg was removed, whereas the insoluble pellet was further homogenized in extraction buffer containing high salt (1 M NaCl in 10 mM Tris pH 7.4). After centrifugation at 27,000 xg (30 min) the supernatant was collected to yield the high salt extractable pool of proteins. Immunoprecipation of laminin was carried out using antibodies specific to laminin. The same amount of radioactivity (TCA-precipitable) was taken from the high salt extract of the various groups (2x10$^6$ cpm). The binding was carried out in solution containing 0.5% Np-40 and 0.1% SDS for 2 h at room temperature. The formed complexes were precipitated by the addition of 10% fixed Staphylococcus aureus suspended in buffer containing 140 mM NaCl, 10 mM Tris pH 7.4, 5 mM EDTA and 0.5% Np-40, followed by centrifugation at 2000 xg.
The pellet was collected, washed and finally suspended in 74 μl of electrophoresis sample buffer, heated to 100°C and centrifuged at 15,000 xg for 3 min and electrophoresed in SDS-PAGE (5% acrylamide with 3.5% stacking gel). Labeled molecular weight markers were electrophoresed in these conditions (not shown). Figure shows three slots representing i) immunoprecipitates with laminin antibodies of high salt extract from nontreated cells (1); CM-treated cells (2); and cells which were treated with boiled CM (3). Arrows on the right point to the two polypeptides which migrated very close to the nonlabeled purified laminin derived from mouse EHS sarcoma.

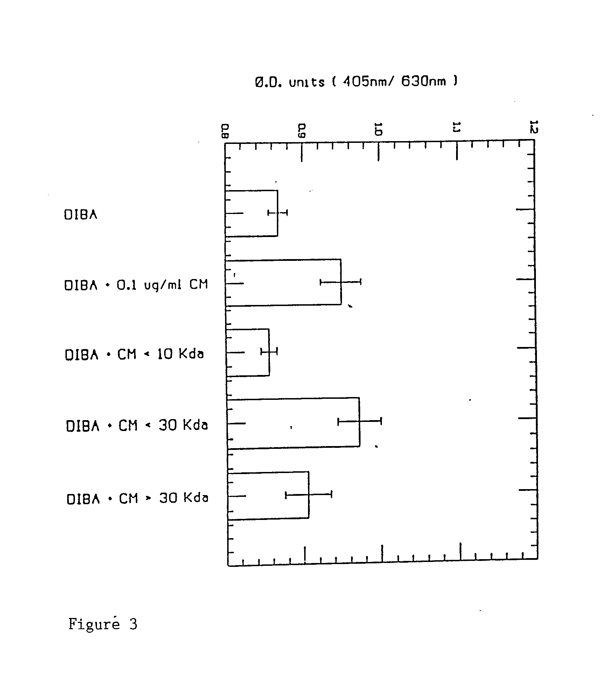

Fig. 3 illustrates how laminin immunoreactive sites in C-6 glioma cells are stimulated by a fraction of the CM having MW between 10 kDa and 30 kDa. Cells were grown in microtiter plates (96 cells). When the cells reached confluency the media was changed and was supplemented with CM (0.1 μg protein/ml) or with the fractionated CM according to the experimental design. The fractionated CM was achieved by first centrifuging through centricon -30 (cut off 30 kDa) and further centrifugation of the filtrate through centricon-10 (cut off 10 kDa). DMEM was added to each fraction in order to get the original volume. The cell-containing plates were centrifuged for 3 min at 1000 xg in an International centrifuge Sorvall GLC-3 and then washed in Tris buffer (0.1 M Tris, pH 7.4, 150 mM NaCl). Each wash involved centrifugation. Nonspecific sites were blocked by BSA (1%) for 30 min at 37°C. Rabbit antibodies directed against laminin at the appropriate dilutions were applied for 45 min. The cells were then washed twice with the Tris buffer and treated with the second antibodies (goat anti-rabbit, IgG conjugated with horseradish peroxidase (50 ul were added to each well for 1-3 h incubation at 37°C or overnight at 4°C). Cells were washed, and 100 ul of substrate (2,2'azino-di-(3-ethylbenzthiazoline sulphonate, ABTS) was added to each well. Absorbance was recorded in a Titertek Multiskan MMC at 405 nm with a reference wavelength of 630 nm.

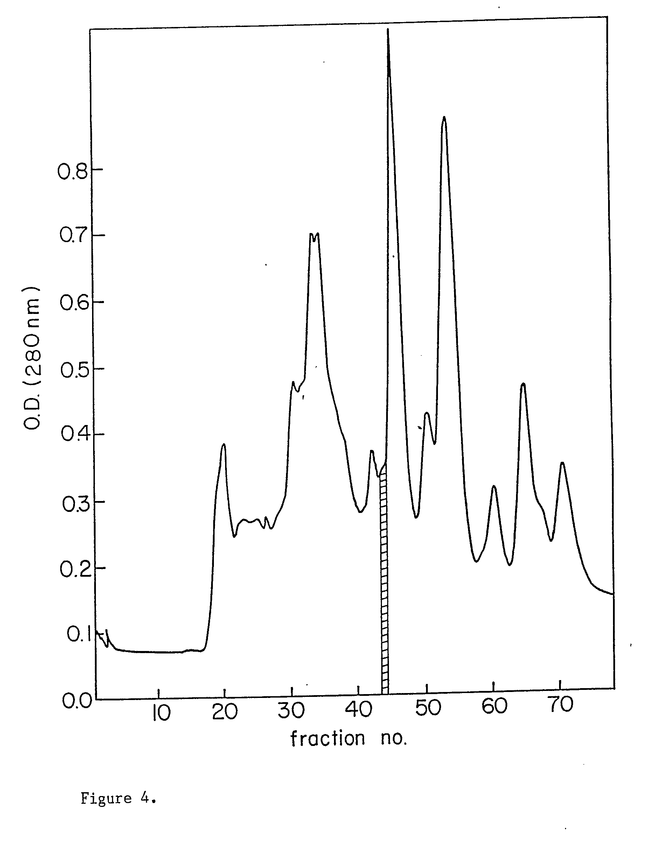

Fig. 4 illustrates how the fractionation of conditioned media for obtaining purified glial modulating (activating) factors is carried out. Gel filtration: Media conditioned by injured fish optic nerves (200 μg/ml) is loaded onto a superose-6 column (Pharmacia). Fractions of 400 ml are collected in phosphate buffer (0.03 M, pH 7.4). Aliquots from each fraction are then taken and applied to C-6 glioma cell cultures, to assay for laminin production, and accumulation by ELISA and immunofluorescence. Finally, confirmation of activity is achieved by pulse labeling with [$^{35}$S]-methionine and immunoprecipitation with anti-laminin antibodies. The labelled bar indicates the peak of activity.

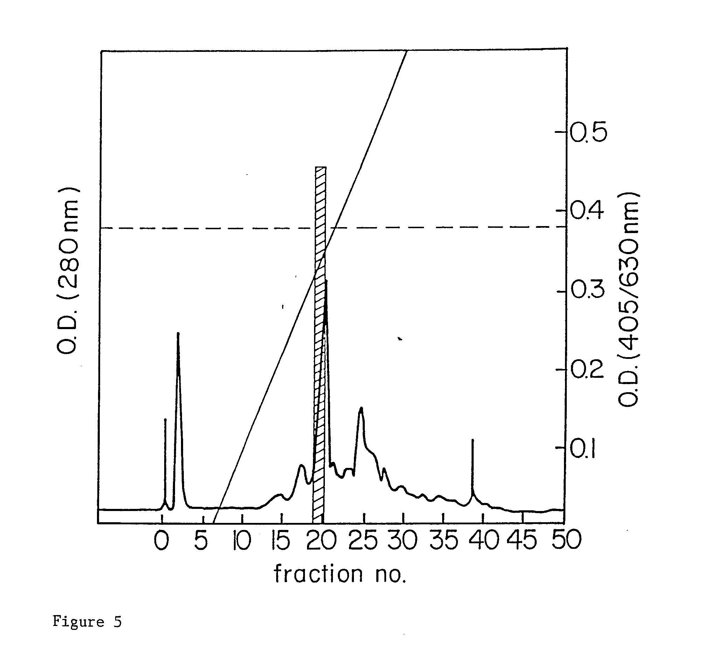

Fig 5 illustrates the use of a Mono-Q anion exchange column (Pharmacia) designed for FPLC for ion exchange. The fractions corresponding to the peak of activity after the superose fractionation are pooled, concentrated, and applied to the Mono-Q column. Elution is carried out in Tris buffer (20 mM, pH 7.2), in a continuous gradient of NaCl (0-0.5 M). Each fraction is then tested for activity. The peak of recovered activity is shown by the bar.

The example illustrates the invention.

Example

Figure 1 shows the immunoreactivity of laminin in the glioma cells by immunofluorescence. The immunoreactivity was elevated in CM-treated cells (Fig. 1A,B), as compared to the CM-free cells (Fig. 1D) or to cells treated with 10% FCS (Fig. 1C). Ten percent FCS was found as optimal, relative to 1% and 5% based on cell proliferation. The concentrations tested were 0.01-10 μg/ml in 10-fold increments. The optimal effect was observed when CM was applied at a concentration of 0.1 μg/ml protein. As shown in Fig. 1A, at 1 μg/ml the level of laminin immunoreactive sites was lower. This concentration-dependence was observed also by ELISA and by immunoprecipitation techniques (not shown). The immunoreactive protein in the glioma cells was verified as laminin by immunoprecipitation. The cells were metabolically labeled with [$^{35}$S]-methionine, followed by immunoprecipitation from a high extract prepared from the cells using laminin-specific antibodies. As shown in Figure 2, in both CM-treated (Fig. 2A, lane 2) and nontreated (Fig. 2A, lane 1) cells, there were two polypeptides (marked by arrows) migrating similarly to the purified laminin from Engelbreth-Holmswarm sarcoma (EHS). Based on the observation that the bands were obtained with anti-laminin antibodies and comigrated with laminin polypeptides, it is possible to conclude that the extracted and precipitated protein is laminin. Immunoprecipitation from the high salt extractable pool of proteins revealed that 0.39% of this pool of proteins are laminin in nontreated cells.

The immunobands of laminin exhibited a higher level of labeling in high salt extractable proteins of CM-treated cells (Fig. 2, lane 2) than in that of nontreated cells (Fig. 2, lane 1). In view of the fact that equal amounts of radioactivity (TCA-precipitable) were taken for the immunoprecipitation, this higher level of labeling represents true elevation of laminin within the pool of high salt extractable proteins of CM-treated cells. The level of labeled laminin was 6-fold higher in high salt extracts of CM-treated cells than in nontreated cells, based on densitometric scanning of the gel shown in Figure 2. Analysis of labeled proteins in the pool obtained by high salt extraction, revealed only 20% elevation in general labeling in CM-treated cells.

This CM-induced elevation of laminin was not unique to the pool of high salt extractable proteins. Its level increases also in the medium, in which the cells were grown. This indicates that the CM causes also an increase in the level of laminin secreted into the media.

Another matrix protein which exhibited a similar pattern of CM-induced increased labeling within the high salt extractable proteins and the proteins secreted into the media was fibronectin. Staphylococcus aureus is known to bind fibronectin specifically (Clark, R.A.F., Nielsen, L.D., Howell, D.N. and Folkvoord, J.M. J. Cell Biochem. 28 (1985) 127-141. Immunoprecipitation of the secreted proteins using either anti-fibronectin antibodies or Staphylococcus aureus without primary antibodies, revealed on gel electrophoresis a protein which comigrated with fibronectin. Moreover, this band showed a higher intensity in CM-treated cells than in nontreated cells.

Heat treated CM had apparently no effect on either laminin (Fig. 2, lane 3), suggesting that a proteinaceous component within the CM is responsible for activation.

In preliminary studies towards isolation of the active substance(s), we fractionated the CM by microconcentrators with a 10,000 and 30,000 MW cut off. The appropriate fractions were applied to the cells and the activity was examined by ELISA. As shown in Figure 3, most of the activity was found in a fraction of CM having a MW between 10 to 30 kDa.

This study shows that media conditioned by fish optic nerves have the capacity to modulate glial cell behavior in vitro. The net modulation is a general but moderate elevation in the level of newly synthesized proteins extracted by high salt. This average may result from much higher elevation of specific proteins, as was shown in this study with respect to laminin and fibronectin. Since these two proteins represent less than 2% of the total pool of extractable proteins, there should be additional proteins which show CM-induced increase in their level.

We have already shown that soluble substances, that originate from growing nerves that induce a regeneration like response in retinas, when applied to adult rabbit optic nerves cause an increased appearance of laminin in the extracellular matrix of the adult mammalian injured nerves (Zak, N., Harel, A., Bawnik, Y., Benbasat, S., Vogel, Z. and Schwartz, M. Brain Res. 408 (1987) 263-266). It has been proposed that the continuous expression of laminin by astrocytes is a prerequisite for axonal growth and regeneration in adult CNS (Liesi, P. EMBO J. 4 (1985) 2505-2511). After optic nerve crush in goldfish, laminin distribution becomes more wide-spread (or diffuse) and intense, especially in regions distal to the crush site (Hopkins, J.M., Ford-Holevinski, T.S., McCoy, J.P. and Agranoff, B.W. J. Neurosci. 5 (1985) 3030-3038; Liesi, P. EMBO J. 4 (1985) 2505-2511). Laminin appears in immature brain cells during CNS development and its presence coincides with phases of neural migration (Liesi, P., Dahl, D. and Vaheri, A. J. Cell Biol. 96 (1983) 920-924; Liesi, P., Dahl, D. and Vaheri, A. J. Neurosci. Res. 11 (1984) 241-251). In adult brain laminin has been detected only in association with capillary walls and meningeal structures in contrast to its widespread distribution in the peripheral nervous system (Bunge, M.B., Williams, A.K., and Wood, P.M. Dev. Biol. 92 (1982) 449-460; Cornbrooks, C.J., Carey, D.J., McDonald, J.A., Timpl, R. and Bunge, R.P. Proc. Natl. Acad. Sci. USA 80 (1983) 3850-3854; McCloon, S.C. Exp. Neurology 91 (1986) 613- 621). Thus, while in vivo adult astrocytes do not produce laminin, astrocytes of developing rat like Schwann cells, were shown in primary cultures to produce and deposit laminin into the extracellular matrix (Liesi, P., Dahl, D. and Vaheri, A. J. Cell Biol. 96 (1983) 920-924). However, in vivo mature astrocytes do produce laminin for short periods after injury in some instances (Bernstein, J.J., Getz, R., Jefferson, M. and Kelemen, M. Brain Res. 327 (1985) 135-141; Liesi, P., Kaakkola, S., Dahl, D. and Vaheri, A. EMBO J. 3 (1984) 683-686), but not in all tested nerves (McCloon, S.C. Exp. Neurology 91 (1986) 613-621).

The combined application of this factor and the use of low energy laser irradiation as a way to delay the degenerative response resulted in an extensive morphological regeneration, not observed before. It is believed that the results observed in this study are also applicable to primary cells of adult mammalian CNS. The results obtained indicated that the glial cells in the CNS, if properly stimulated, could make a similar contribution to successful growth as the Schwann cells in peripheral nerves. It is possible that soluble substances originating from regenerating nerves supply their own cells in the appropriate activating molecules. In nonregenerating nerves, these molecules seem to provide exogenous factors which, when applied to injured adult CNS cause, for example, a widespread distribution of laminin (Zak, N., Harel, A., Bawnik, Y., Benbassat, S., Vogel, Z. and Schwartz, M. Brain Res. 408 (1987) 263-266). This seems to be the first step towards circumventing the inability to regenerate.

## Claims

1. A water soluble factor which is obtainable from a regenerating optical nerve of a fish by subjecting material released from said regenerating optical nerve to column chromatography, and which activates target cells, preferably glial cells, to produce extracellular proteins and mainly laminin.

2. The factor according to claim 1, which modifies the response of glial cells to injuries of the mammalian central nervous system, and enhances the syntheses and accumulation of various extracellular proteins, mainly of laminin.

3. The factor according to claim or 2, which is a negatively charged protein which exhibits one peak of an apparent MW of 10,000 ± 300 on a sieving type column, and which when subjected to chromatography on a Mono-Q type column exhibits two peaks, one eluted at about 0.16 M sodium chloride, the other at about 0.3 M sodium chloride.

4. A process for the production of the factor of any one of claims 1 to 3 which comprises subjecting material released from regenerating optical nerves of a fish to column chromatography.

5. A factor according to any one of claims 1 to 3 for use as a pharmaceutically active substance.

6. A factor according to any one of claims 1 to 3 for use as a pharmaceutically active substance for inducing the regeneration of nerves of the central nervous system of mammals.

7. A pharmaceutical composition containing a factor according to any one of claims 1 to 3 and optionally a pharmaceutically acceptable carrier and/or diluent.

8. The pharmaceutical composition according to claim 7 for inducing the regeneration of nerves of the central nervous system of mammals.

9. The pharmaceutical composition according to claim 7 or 8 for the combined application with irradiation of the injured site with a low energy He-Ne laser at predetermined intervals.

10. The pharmaceutical composition according to claim 9 wherein the intervals are daily intervals.

Claims for the following Contracting State: GR

1. A water soluble factor which is obtainable from a regenerating optical nerve of a fish by subjecting material released from said regenerating optical nerve to column chromatography, and which activates target cells, preferably glial cells, to produce extracellular proteins and mainly laminin.

2. The factor according to claim 1, which modifies the response of glial cells to injuries of the mammalian central nervous system, and enhances the syntheses and accumulation of various extracellular proteins, mainly of laminin.

3. The factor according to claim 1 or 2, which is a negatively charged protein which exhibits one peak of an apparent MW of 10,000 ± 300 on a sieving type column, and which when subjected to chromatography on a Mono-Q type column exhibits two peaks, one eluted at about 0.16 M sodium chloride, the other at about 0.3 M sodium chloride.

4. A process for the production of the factor of any one of claims 1 to 3 which comprises subjecting material released from regenerating optical nerves of a fish to column chromatography.

5. A factor according to any one of claims 1 to 3 for use as a pharmaceutically active substance.

6. A factor according to any one of claims 1 to 3 for use as a pharmaceutically active substance for inducing the regeneration of nerves of the central nervous system of mammals.

7. A factor according to any one of claims 1 to 3 for use as a pharmaceutically active substance for inducing the regeneration of nerves of the central nervous system of mammels in combination with radiation of the injured site with a low energy He-Ne laser at predetermined intervals, preferably at daily intervals.

Claims for the following Contracting State : ES

1. A process for the production of a water soluble factor from a regenerating optical nerve of a fish which activates target cells, preferably glial cells, to produce extracellular protein and mainly laminin, said process comprising subjecting material released from a regenerating optical nerve of a fish to column chromatography.

2. The process according to claim 1, wherein the factor modifies the response of glial cells to injuries of the mammalian central nervous system, and enhances the synthesis and accumulation of various extracellular proteins, mainly of laminin.

3. The process according to claim 1 or 2 wherein said factor is a negatively charged protein which exhibits one peak of an apparent MW of $10,000 \pm 300$ on a sieving type column, and which when subjected to chromatography on a Mono-Q column exhibits two peaks, one eluted at about 0.16 M sodium chloride, the other at about 0.3 M sodium chloride.

Figure 1

Figure 2

Figuré 3

Figure 4.

Figure 5